# EUROPÄISCHE PATENTANMELDUNG

(11) **EP 4 574 261 A1**
(43) Veröffentlichungstag der Anmeldung: **25.06.2025**
(21) Anmeldenummer: 24220870.0
(22) Anmeldetag: 18.12.2024
(51) Int. Cl.: B01L 3/00

(54) **FALTSTREIFEN ZUR HERSTELLUNG EINER VORRICHTUNG FÜR EINEN LATERAL-FLOW-TEST**

(30) Priorität: 18.12.2023 AT 510162023
(71) Anmelder: Tagtron GmbH, 2340 Mödling (AT)
(72) Erfinder: MITTERNDORFER, Fridolin, 4844 Regau (AT); SCHEFZIG, Fabian, 4844 Regau (AT); SCHODERMAYR, Christoph, 4844 Regau (AT)
(74) Vertreter: SONN Patentanwälte GmbH & Co KG

(57) **Zusammenfassung**

Die Erfindung betrifft einen Faltstreifen (1, 1A-F) zur Herstellung einer Vorrichtung (23) für einen Lateral-Flow-Test, aufweisend einen Grundabschnitt (2) und einen Deckabschnitt (3), eine in einem ungefalteten Zustand des Faltstreifens (1, 1A-F) zwischen dem Grundabschnitt (2) und dem Deckabschnitt (3) angeordnete Falzkante (16, 16a-c), wobei der Grundabschnitt (2) einen Befestigungsbereich (4) zur Befestigung eines Teststreifens (5) aufweist, wobei der Deckabschnitt (3) ein Sichtfenster (9) und ein Applikationsfenster (8) aufweist, und wobei in einem gefalteten Zustand des Faltstreifens (1, 1A-F) ein erster Teil (10a) des Befestigungsbereichs (4) zwischen dem Grundabschnitt (2) und dem Applikationsfenster (8) und ein zweiter Teil (10b) des Befestigungsbereichs (4) zwischen dem Grundabschnitt (2) und dem Sichtfenster (9) angeordnet ist.

## Beschreibung

Die Erfindung betrifft einen Faltstreifen zur Herstellung einer Vorrichtung für einen Lateral-Flow-Test. Weiters betrifft die Erfindung ein Verfahren zur Herstellung der Vorrichtung aus dem Faltstreifen, einen Testbogen zur Herstellung von zwei oder mehr Vorrichtungen, sowie die Vorrichtung selbst.

Lateral-Flow-Tests werden zum qualitativen Nachweis von Stoffen verwendet, etwa mittels einer Antigen-Antikörper-Reaktion. Sie können auf dem Prinzip der Dünnschichtchromatographie und der Immunmarkierung beruhen. Insbesondere während der COVID-19-Pandemie wurden Lateral-Flow-Tests häufig eingesetzt, um den Nachweis einer Infektion mit SARS-CoV-2 rasch festzustellen. Lateral-Flow-Tests werden auch zum Nachweis einer Schwangerschaft, einer Drogenaufnahme oder von Influenza eingesetzt.

In AT 17585 U1 wird eine Vorrichtung beschrieben, die ein rohrförmiges Gehäuse mit einer inneren Schicht aus einem flüssigkeitsresistenten Karton und einer äußeren Schicht aus einem gerollten Karton aufweist. Eine Speichelprobe wird der Vorrichtung zugeführt, indem die Vorrichtung in den Mund genommen wird.

Im Wesentlichen quaderförmige Vorrichtungen für einen Lateral-Flow-Test offenbaren beispielsweise WO 2013/158504 A1, WO 2019/215199 A1 und WO 2014/134033 A1. Mittels eines Probenahme-Tupfers wird eine Probe umfassend z.B. einen Nasen- oder Rachenabstrich auf einen im Gehäuse aufgenommenen Teststreifen appliziert. Bei diesen Vorrichtungen ist das Gehäuse üblicherweise aus einem Kunststoff gefertigt und wird mittels Spritzgießen hergestellt.

Die Herstellung des Gehäuses im Spritzgießverfahren ist aufwändig. Einerseits besteht das Gehäuse zumeist aus zwei Gehäuseteilen, die separat spritzgegossen werden, was den Einsatz von zwei Formwerkzeugen mit unterschiedlicher Geometrie erfordert. Andererseits sind aufgrund des komplexen, Gehäuseaufbaus geometrisch aufwändige Formwerkzeuge notwendig. Insbesondere müssen Elemente zur nachfolgenden Verbindung der Gehäuseteile vorgesehen werden, sowie Hinterschneidungen, um den Teststreifen im Gehäuse zu befestigen. Daraus resultiert insgesamt eine energie- und kostenintensive Herstellung der Vorrichtung.

Es ist eine Aufgabe der Erfindung, eine Vorrichtung für einen Lateral-Flow-Test in einem einfacheren, nachhaltigeren Verfahren herzustellen.

Die Erfindung betrifft einen Faltstreifen zur Herstellung einer Vorrichtung für einen Lateral-Flow-Test, aufweisend
einen Grundabschnitt und einen Deckabschnitt,
eine in einem ungefalteten Zustand des Faltstreifens zwischen dem Grundabschnitt und dem Deckabschnitt angeordnete Falzkante,
wobei der Grundabschnitt einen Befestigungsbereich zur Befestigung eines Teststreifens aufweist,
wobei der Deckabschnitt ein Sichtfenster und ein Applikationsfenster aufweist, und
wobei in einem gefalteten Zustand des Faltstreifens ein erster Teil des Befestigungsbereichs zwischen dem Grundabschnitt und dem Applikationsfenster und ein zweiter Teil des Befestigungsbereichs zwischen dem Grundabschnitt und dem Sichtfenster angeordnet ist.

Aus dem Faltstreifen lässt sich in einfacher, kostengünstiger Weise durch entsprechendes Falten mittels der Falzkante eine Vorrichtung für einen Lateral-Flow-Test herstellen. Da das Gehäuse keinen Einfluss auf die Probennahme hat und einen ungestörten Lateral-Flow-Test ermöglichen soll, ist die Art des Lateral-Flow-Tests selbst für die vorliegende Erfindung von geringer Bedeutung. Jeder am Markt übliche, insbesondere zertifizierte, Teststreifen kann zum Einsatz kommen. Die Erfindung trägt somit aufgrund des niedrigeren Energie- und Kosteneinsatzes bei der Herstellung des Faltstreifens sowie nachfolgend bei der Herstellung der Vorrichtung maßgeblich zur Nachhaltigkeit bei.

Im ungefalteten Zustand des Faltstreifens ist die Falzkante zwischen dem Grundabschnitt und dem Deckabschnitt angeordnet. Unter dem ungefalteten Zustand des Faltstreifens wird ein Zustand verstanden, in welchem die Abschnitte nebeneinanderliegend, z.B. in einem Winkel zueinander von im Wesentlichen 180°, im Vergleich zum gefalteten Zustand, angeordnet sind.

Die Falzkante teilt den Faltstreifen in zusammenklappbare Faltteile, die zumindest durch den Grundabschnitt und den Deckabschnitt gebildet werden. Die Falzkante kann eine Vertiefung aufweisen. Dadurch kann der Faltstreifen leicht gefaltet werden. Die Vertiefung kann eine Rille und/oder eine Nut aufweisen. Zusätzlich kann auch eine optische Markierung vorhanden sein. Die Abschnitte des Faltstreifens können mittels der Falzkante einfach gefaltet und übereinanderliegend angeordnet werden. Unter dem gefalteten Zustand des Faltstreifens wird ein Zustand verstanden, in welchem die Abschnitte mittels der Falzkante derart gefaltet wurden, dass sie übereinanderliegend und parallel zueinander angeordnet sind, und wobei der Teststreifen, wenn vorhanden, zwischen dem Grundabschnitt und dem Deckabschnitt angeordnet ist.

Das Sichtfenster und das Applikationsfenster sind als Ausnehmungen im Deckabschnitt ausgebildet. Diese Ausnehmungen können beispielsweise durch Stanzen oder Lasern erhalten werden. Hierdurch kann eine hohe Präzision erreicht werden. Durch das Sichtfenster und das Applikationsfenster sind im gefalteten Zustand des Faltstreifens ein erster Teil und ein zweiter Teil des Befestigungsbereichs von außen für einen Anwender sichtbar bzw. zugänglich. Wenn ein Teststreifen am Befestigungsbereich befestigt ist, sollen das Applikationsfenster und das Sichtfenster im gefalteten Zustand des Faltstreifens Zugang bzw. Sicht zu einem Applikationsbereich und einem Ergebnisbereich des Teststreifens ermöglichen.

Der Faltstreifen kann einen Teststreifen aufweisen, der am Befestigungsbereich befestigt ist. Der Teststreifen kann einen rechteckigen Grundriss haben. Der Teststreifen kann einen Träger aufweisen, mit welchem der Teststreifen am Befestigungsbereich befestigt wird. Auf dem Träger kann ein Laufstreifen zur Aufnahme und Messung einer Probe (z.B. eine Flüssigkeit, insbesondere ein Nasen- oder Rachenabstrich oder eine Speichel-, Serum- oder Blutprobe) befestigt sein. Der Laufstreifen kann einen Applikationsbereich aufweisen, auf welchen die Probe aufgebracht werden kann, und einen Ergebnisbereich, in welchem sich im Falle eines durchgeführten Tests eine Kontrolllinie (C) ausbilden kann, und im Falle eines positiven Tests weiters eine Testlinie (T). Das Verfahren zur Durchführung des Lateral-Flow-Tests ist im Stand der Technik, insbesondere seit der Covid-19-Pandemie, hinlänglich bekannt (siehe z.B. WO 2020/237308 A1, WO 2021/231659 A1 oder US 2021/325383 A1.

Der Teststreifen wird am Befestigungsbereich derart befestigt, dass ein erster Teil des Teststreifens im gefalteten Zustand des Faltstreifens zwischen dem Grundabschnitt und dem Applikationsfenster angeordnet ist, und dass ein zweiter Teil des Teststreifens im gefalteten Zustand des Faltstreifens zwischen dem Grundabschnitt und dem Sichtfenster angeordnet ist. Dabei weist der erste Teil des Teststreifens den Applikationsbereich auf und der zweite Teil des Teststreifens den Ergebnisbereich.

Der Teststreifen ist für den erfindungsgemäßen Faltstreifen optional. Der Faltstreifen kann beispielsweise ohne den Teststreifen hergestellt und vermarktet werden, wobei die Kunden Hersteller von Vorrichtungen für einen Lateral-Flow-Test sein können und vor dem Falten des Faltstreifens einen für sie gewünschten Teststreifen am Befestigungsbereich befestigen können. Diese Vorrichtungen können dann an Endkunden, die den Test durchführen, geliefert werden. Um den Test an einer zu untersuchenden Probe durchzuführen, ist natürlich der Teststreifen notwendig.

Der Faltstreifen kann einen im Wesentlichen rechteckigen Grundriss haben. Dies kann nicht nur die Herstellung des Faltstreifens selbst vereinfachen, sondern auch eine exakte, bündig übereinanderliegende Anordnung der Abschnitte im gefalteten Zustand vereinfachen.

Die Falzkante kann normal zu einer Längsseite des Faltstreifens sein. Dies kann einen vertikalen Schichtaufbau beim Falten erleichtern, insbesondere, wenn der Faltstreifen mehr als zwei zu faltende Abschnitte aufweist. Wenn mehr als zwei zu faltende Abschnitte vorhanden sind, kann zwischen jeweils zwei benachbarten (d.h. unmittelbar hintereinander angeordnete) Abschnitten eine Falzkante vorhanden sein. Jede dieser Falzkanten kann normal zu einer Längsseite des Faltstreifens sein.

Eine Längsseite des Teststreifens kann parallel zu einer Längsseite des Faltstreifens sein. Hierdurch können die Abmessungen des Faltstreifens möglichst gut ausgenutzt werden, insbesondere, wenn der Faltstreifen einen im Wesentlichen rechteckigen Grundriss hat.

Der Teststreifen, insbesondere der Träger des Teststreifens, kann mit einem Klebstoff und/oder mit einem doppelseitigen Klebeband am Befestigungsbereich befestigt sein. Dann kann er beim nachfolgenden Falten nicht verrutschen und seine Position beibehalten, sodass sichergestellt ist, dass der erste Teil und der zweite Teil des Teststreifens für einen Anwender von außen durch das Applikationsfenster und das Sichtfenster sichtbar bzw. zugänglich sind.

Der Befestigungsbereich des Grundabschnitts kann ein Distanzelement aufweisen. Dann kann der Teststreifen, insbesondere der Träger des Teststreifens, am Distanzelement befestigt werden. Hierdurch kann die Gefahr eines Kontakts zwischen einer auf den Teststreifen aufgebrachten Probe und dem Grundabschnitt reduziert werden. Das Distanzelement kann beispielsweise unter Verwendung von Prägen, Spritzgießen, Hochziehen und/oder Tiefziehen im Faltstreifen vorgesehen werden. Das Distanzelement kann zwei oder mehr Distanzstege aufweisen. Hierdurch kann der Teststreifen auf dem Distanzelement im Gleichgewicht gehalten werden. Die Distanzstege können parallel zueinander und parallel zu einer Längsseite des Faltstreifens sein. In diesem Fall kann das Vorsehen von zwei Distanzstegen ausreichend sein. Alternativ können die Distanzstege parallel zueinander und parallel zu einer Breitseite des Faltstreifens sein. Dann sollte eine größere Anzahl an Distanzstegen vorgesehen werden, z.B. vier bis zehn, oder vier bis acht. Damit kann eine gute Stabilisierung und Befestigung des Teststreifens erreicht werden.

Der Faltstreifen kann einen Karton und/oder einen Kunststoff aufweisen. Der Faltstreifen kann Zellstoff und/oder Zellulose enthalten. Hierdurch kann der Faltstreifen mittels üblicher Verfahren, insbesondere unter Verwendung von Schneiden, Stanzen, Prägen, Lasern, Hochziehen, Tiefziehen, Spritzgießen und/oder Drucken, einfach hergestellt werden. Derartige Materialien sind umweltverträglich und ermöglichen eine günstige und nachhaltige Herstellung sowie Entsorgung der Vorrichtung.

Weist der Faltstreifen einen Karton auf, kann dieser ein Flächengewicht von 200 g/m² oder darüber aufweisen, insbesondere von 300 g/m² oder darüber. Hierdurch kann eine gute Steifigkeit und Festigkeit bereitgestellt werden, und kann eine Beschädigung des Faltstreifens (z.B. Riss, Knick) bei der Herstellung des Faltstreifens bzw. nachfolgend bei der Herstellung der Vorrichtung vermieden werden. Durch dieses Flächengewicht kann eine Dicke der Abschnitte bereitgestellt werden, die gut geeignet ist, ein stabiles Gehäuse für einen Teststreifen zu bilden.

Der Faltstreifen kann aus einem Kunststoff bestehen. Dann ist eine einfache Herstellung des Faltstreifens mittels üblicher kunststofftechnischer Verfahren möglich, insbesondere mittels Spritzgießen. Während in den im Stand der Technik üblichen Verfahren Gehäuseteile separat spritzgegossen und danach verbunden werden, was die Verwendung von zumindest zwei unterschiedlichen Formwerkzeuge erfordert, ist nur ein einziges Formwerkzeug notwendig, um den Faltstreifen gemäß der Erfindung herzustellen. Weiters kann dieses Formwerkzeug eine geometrisch vergleichsweise einfache Kavität aufweisen, insbesondere brauchen keine Elemente zur Verbindung von Gehäuseteilen oder Hinterschneidungen zur Befestigung des Teststreifens vorgesehen zu werden. Die Verfahrensführung beim Spritzgießen vereinfacht sich dadurch erheblich.

Eine im gefalteten Zustand außenliegende Seite des Grundabschnitts und/oder des Deckabschnitts kann wasserabweisend sein. Wird der Faltstreifen einer feuchten bzw. nassen Umgebung ausgesetzt, kann eine Beschädigung des Faltstreifens (z.B. Aufweichen, Anlösen) dadurch vermieden werden. Diese außenliegende Seite kann beschichtet sein, beispielsweise mit einem hydrophoben Kunststoff, insbesondere mit einem Polyolefin. Eine Beschichtung kann vorteilhaft sein, wenn der Faltstreifen einen Karton umfasst. Der Faltstreifen kann auch aus einem hydrophoben Kunststoff bestehen. Dann braucht keine zusätzliche Beschichtung vorgesehen zu werden. Alternativ oder zusätzlich kann eine im gefalteten Zustand innenliegende Seite des Grundabschnitts und/oder des Deckabschnitts wasserabweisend sein. Dadurch können Störungen bei der Durchführung der Messung vermieden werden.

Der Faltstreifen kann eine unebene Seitenfläche aufweisen. Insbesondere kann die Seitenfläche Zähne, Kerben, und/oder Zacken aufweisen. Daraus wird ersichtlich, dass der Faltstreifen durch Abtrennen von einem weiteren Faltstreifen mittels einer Perforationslinie erhalten worden ist. Unter der Seitenfläche wird eine Fläche des Faltstreifens verstanden, die im gefalteten Zustand normal zu der Seite des Grundabschnitts ist, mit welcher der Teststreifen verbunden ist.

Der Faltstreifen kann weiters einen Distanzabschnitt aufweisen, der im gefalteten Zustand des Faltstreifens zwischen dem Grundabschnitt und dem Deckabschnitt angeordnet ist. Der Distanzabschnitt kann einen Rahmen aufweisen, der vorzugsweise eingerichtet ist, im gefalteten Zustand des Faltstreifens beabstandet zum Teststreifen, insbesondere zum Laufstreifen des Teststreifens, zu sein. Damit ist gemeint, dass Innenkanten des Rahmens eingerichtet sind, im gefalteten Zustand des Faltstreifens beabstandet zum Teststreifen zu sein. Der Teststreifen kann dabei zumindest teilweise innerhalb einer durch den Rahmen des Distanzabschnitts gebildeten Ausnehmung angeordnet sein (d.h. im bestimmungsgemäßen Zustand des gefalteten Faltstreifens sind der Rahmen und der Teststreifen dann zumindest teilweise auf gleicher Höhe angeordnet). Durch den Rahmen kann insbesondere eine rechteckige Ausnehmung im Distanzabschnitt gebildet werden.

Vorzugsweise ist der Rahmen eingerichtet, im gefalteten Zustand des Faltstreifens beabstandet zu einer Längsseite des Teststreifens zu sein, insbesondere beabstandet zum Laufstreifen. Dann wird verhindert, dass bei der Verwendung des Lateral-Flow-Tests Flüssigkeit vom Teststreifen, insbesondere vom Laufstreifen, auf den Distanzabschnitt, und gegebenenfalls auch wieder zurück, übertreten kann.

Der Rahmen kann durch ein umlaufendes Profil gebildet sein. Der Rahmen kann vier Seiten aufweisen, die eingerichtet sind, im gefalteten Zustand des Faltstreifens beabstandet zum Teststreifen zu sein. Die vier Seiten des Rahmens können zwei Breitseiten und zwei Längsseiten sein. Die Breitseiten des Rahmens können auch nur durch die Falzkanten ausgebildet sein. Für eine höhere Stabilität können die Breitseiten aber auch breiter als die Falzkanten ausgeführt sein. Der Rahmen kann z.B. durch Stanzen des Faltstreifens gebildet werden.

Dadurch, dass der Rahmen, und dementsprechend der Distanzabschnitt, eingerichtet ist, im gefalteten Zustand des Faltstreifens beabstandet zum Teststreifen angeordnet zu sein, wird sichergestellt, dass eine auf den Teststreifen aufgebrachte Probe auch am Teststreifen, insbesondere auf dessen Laufstreifen, verbleibt, sodass eine verbesserte Messung durchgeführt werden kann. Würde der Distanzabschnitt den Teststreifen, insbesondere den Laufstreifen, vor allem den Ergebnisbereich, berühren, wäre damit die Gefahr verbunden, dass die Probe auf den Distanzabschnitt wandert und eine verschlechterte Messung durchgeführt wird. Durch den Distanzabschnitt erhöht sich außerdem die Stabilität der aus dem Faltstreifen hergestellten Vorrichtung. Ein Kontakt des Distanzabschnitts zum Träger des Teststreifens kann zwar vorgesehen werden. Vorzugsweise wird für einen seitlichen Kontakt zum Träger aber ein Fixierabschnitt vorgesehen.

Der Distanzabschnitt kann im ungefalteten Zustand des Faltstreifens zwischen dem Grundabschnitt und dem Deckabschnitt angeordnet sein, wobei je eine Falzkante zwischen dem Grundabschnitt und dem Distanzabschnitt sowie zwischen dem Distanzabschnitt und dem Deckabschnitt angeordnet ist. Alternativ kann der Grundabschnitt im ungefalteten Zustand des Faltstreifens zwischen dem Distanzabschnitt und dem Deckabschnitt angeordnet sein, wobei je eine Falzkante zwischen dem Distanzabschnitt und dem Grundabschnitt sowie zwischen dem Grundabschnitt und dem Deckabschnitt angeordnet ist.

Ausführungsformen des Faltstreifens ohne Distanzabschnitt sind zwar möglich, aber weniger bevorzugt. In diesen Ausführungsformen müsste im gefalteten Zustand des Faltstreifens durch den Grund- und/oder Deckabschnitt ein Hohlraum gebildet werden, in den ein Teststreifen zwischen dem Grundabschnitt und dem Deckabschnitt aufgenommen werden kann. Ein solcher Hohlraum kann z.B. durch eine Vertiefung eines oder beider Abschnitte (ausgewählt aus Grund- und Deckabschnitt) gebildet werden. Ein entsprechender Hohlraum für den Teststreifen kann aber bei der erfindungsgemäßen Falttechnik einfacher durch das Vorsehen eines Distanzabschnitts hergestellt werden (hierbei kann der Teststreifen in der/den durch den Rahmen des Distanzabschnitts und/oder in der durch den Rahmen des Fixierabschnitts gebildeten Ausnehmung(en) vorgesehen werden). Dann können der Grund- und Deckabschnitt planar sein, was die Herstellung des Faltstreifens vereinfacht.

Der Faltstreifen kann weiters einen Fixierabschnitt aufweisen, der im gefalteten Zustand des Faltstreifens zwischen dem Grundabschnitt und dem Deckabschnitt angeordnet ist. Der Fixierabschnitt kann einen Rahmen aufweisen, der vorzugsweise eingerichtet ist, im gefalteten Zustand des Faltstreifens zumindest teilweise am Teststreifen, insbesondere am Träger des Teststreifens, anzuliegen. Der Teststreifen kann dabei zumindest teilweise innerhalb einer durch den Rahmen des Fixierabschnitts gebildeten Ausnehmung angeordnet sein (d.h. im bestimmungsgemäßen Zustand des gefalteten Faltstreifens sind der Rahmen und der Teststreifen dann zumindest teilweise auf gleicher Höhe angeordnet). Der Rahmen des Fixierabschnitts kann z.B. durch Stanzen des Faltstreifens gebildet werden. Der Rahmen kann durch ein umlaufendes Profil gebildet sein.

Der Rahmen des Fixierabschnitts kann rechteckig sein, aber auch andere Rahmengeometrien sind denkbar. Beispielsweise kann der Rahmen vier Seiten aufweisen. Die vier Seiten des Rahmens können zwei Breitseiten und zwei Längsseiten sein. Die Breitseiten des Rahmens können auch nur durch die Falzkanten ausgebildet sein. Für eine höhere Stabilität können die Breitseiten aber auch breiter als die Falzkanten ausgeführt sein.

Zumindest ein Teil einer der Seiten des Rahmens, insbesondere ein Teil einer der Längsseiten oder jeweils ein Teil beider Längsseiten, kann eingerichtet sein, im gefalteten Zustand des Faltstreifens am Teststreifen, insbesondere am Träger des Teststreifens, anzuliegen. An der Seite des Rahmens, die im gefalteten Zustand des Faltstreifens nicht am Teststreifen anliegt, ist der Rahmen dementsprechend beabstandet zum Teststreifen angeordnet. Insbesondere kann der Rahmen im gefalteten Zustand des Faltstreifens eingerichtet sein, im Ergebnisbereich des Teststreifens beabstandet zum Teststreifen zu sein, und in endnahen Bereichen des Teststreifens (d.h. außerhalb des Ergebnisbereichs, und vorzugsweise auch außerhalb des Applikationsbereichs) am Teststreifen, insbesondere am Träger, anzuliegen. Dann kann die Probe nicht auf den Fixierabschnitt wandern, und kann eine bessere Messung durchgeführt werden.

Vorzugsweise weist eine Längsseite des Rahmens des Fixierabschnitts einen Teil auf, der breiter ist als eine Längsseite des Rahmens des Distanzabschnitts, und/oder vorzugsweise ist die Ausnehmung, die durch den Rahmen des Fixierabschnitts gebildet wird, an einem Teil ihrer Längsseite schmäler als die durch den Rahmen des Distanzabschnitts gebildete Ausnehmung an ihrer (gesamten) Längsseite. Dieser breite Teil der Längsseite des Rahmens des Fixierabschnitts kann eingerichtet sein, im gefalteten Zustand des Faltstreifens am Teststreifen, insbesondere am Träger des Teststreifens, anzuliegen. Im gefalteten Zustand des Faltstreifens kann dann der Rahmen des Fixierabschnitts den Teststreifen fixieren, vorzugsweise formschlüssig. Dadurch kann der Teststreifen im gefalteten Zustand des Faltstreifens auch dann nicht verrutschen, falls er sich von seiner Befestigung am Befestigungselement löst (z.B. aufgrund von Versagen einer Klebeverbindung).

Vorzugsweise ist der breite Teil des Rahmens des Fixierabschnitts in einem endnahen Bereich des Befestigungsbereichs angeordnet (im gefalteten Zustand des Faltstreifens). Wenn der Faltstreifen den Teststreifen aufweist, ist der breite Teil des Rahmens, an dem der Teststreifen anliegt, vorzugsweise in einem endnahen Bereich des Teststreifens angeordnet, insbesondere in einem Bereich des Teststreifens außerhalb des Ergebnisbereichs, und vorzugsweise auch außerhalb des Applikationsbereichs (im gefalteten Zustand des Faltstreifens). Dann kann der Rahmen des Fixierabschnitts im Ergebnisbereich beabstandet zum Teststreifen sein, sodass die Probe nicht auf den Fixierabschnitt wandern kann.

Der breite Teil der Längsseite des Rahmens des Fixierabschnitts korrespondiert vorzugsweise mit einem schmalen Teil der Längsseite der durch den Rahmen des Fixierabschnitts gebildeten Ausnehmung. Dann kann der Fixierabschnitt einen im Wesentlichen rechteckigen Grundriss haben. Insbesondere kann der Fixierabschnitt dann im gefalteten Zustand des Faltstreifens bündig mit den anderen Abschnitten des Faltstreifens sein, um eine quaderförmige Vorrichtung herzustellen.

Der Rahmen des Fixierabschnitts kann auch mehrere vergleichsweise breite Teile (wie vorgehend beschrieben) aufweisen, wobei zwei oder mehr breite Teile an einer Längsseite angeordnet sein können, oder auch jeweils zwei oder mehr breite Teile an beiden Längsseiten angeordnet sein können. Beispielsweise kann eine Längsseite des Rahmens an ihrer Innenkante (d.h. an der dem Befestigungsbereich zugewandten Kante) wellenförmig sein. Die wellenförmige Längsseite kann zwei oder mehr Wellenberge aufweisen, beispielsweise zwei bis sechs, insbesondere vier. Jeder Wellenberg stellt einen breiten Teil der Längsseite des Rahmens des Fixierabschnitts dar.

Bevorzugt weist der Rahmen des Fixierabschnitts an beiden Längsseiten jeweils zwei Teile auf, die breiter sind als eine Längsseite des Rahmens des Distanzabschnitts. Diese breiten Teile der Längsseite des Rahmens des Fixierabschnitts sind vorzugsweise in einander entgegengesetzten, endnahen Bereichen des Befestigungsbereichs angeordnet (im gefalteten Zustand des Faltstreifens). Dadurch kann der Teststreifen an seinen beiden endnahen Bereichen gleichmäßig fixiert werden. Somit kann eine Verschiebung oder Verdrehung des Teststreifens bei seiner Fixierung im Fixierabschnitt vermieden werden.

Der Fixierabschnitt kann im ungefalteten Zustand des Faltstreifens zwischen dem Grundabschnitt und dem Deckabschnitt angeordnet sein, wobei je eine Falzkante zwischen dem Grundabschnitt und dem Fixierabschnitt sowie zwischen dem Fixierabschnitt und dem Deckabschnitt angeordnet ist. Alternativ kann der Grundabschnitt im ungefalteten Zustand des Faltstreifens zwischen dem Fixierabschnitt und dem Deckabschnitt angeordnet sein, wobei je eine Falzkante zwischen dem Fixierabschnitt und dem Grundabschnitt sowie zwischen dem Grundabschnitt und dem Fixierabschnitt angeordnet ist.

Der Faltstreifen kann den Grundabschnitt, den Deckabschnitt, den Distanzabschnitt und den Fixierabschnitt aufweisen, wobei im ungefalteten Zustand des Faltstreifens jeweils eine Falzkante zwischen den Abschnitten angeordnet ist. Die Falzkanten können an Breitseiten der Abschnitte vorgesehen sein. Die Anordnung der Abschnitte sollte derart sein, dass im gefalteten Zustand des Faltstreifens der Fixierabschnitt zwischen dem Grundabschnitt und dem Distanzabschnitt angeordnet ist, und dass der Distanzabschnitt zwischen dem Fixierabschnitt und dem Deckabschnitt angeordnet ist. Der Faltstreifen kann dann im gefalteten Zustand eine für eine gute Stabilität erforderliche Dicke aufweisen, der Teststreifen (insbesondere der Träger) kann mit dem Fixierabschnitt gut fixiert werden, und aufgrund der durch das Distanzabschnitt erzielten Beabstandung des Faltstreifens zum Teststreifen (insbesondere zum Laufstreifen) kann sichergestellt werden, dass eine Probe auf dem Teststreifen (insbesondere dem Laufstreifen) verbleibt, sodass eine verbesserte Messung durchgeführt werden kann.

Die Abschnitte können im ungefalteten Zustand des Faltstreifens in der folgenden Reihenfolge nacheinander angeordnet sein: Fixierabschnitt, Grundabschnitt, Distanzabschnitt, Deckabschnitt. Auch eine andere Anordnung kann gewählt werden, solange die obengenannte Reihenfolge der Abschnitte im gefalteten Zustand des Faltstreifens erhalten werden kann, z.B. die Reihenfolge Grundabschnitt, Fixierabschnitt, Distanzabschnitt, Deckabschnitt. Ohne Fixierabschnitt kann die Reihenfolge Grundabschnitt, Distanzabschnitt, Deckabschnitt; oder Distanzabschnitt, Grundabschnitt, Deckabschnitt; oder Grundabschnitt, Deckabschnitt, Distanzabschnitt sein. Durch entsprechende Faltung kann im gefalteten Zustand des Faltstreifens eine der folgenden Schichtungen, von unten nach oben (in der bestimmungsgemäßen Ausrichtung des Faltstreifens), erhalten werden: Grundabschnitt, Fixierabschnitt, Distanzabschnitt, Deckabschnitt; oder Grundabschnitt, Distanzabschnitt, Deckabschnitt.

Je nachdem, welche Anordnung der Abschnitte gewählt wird, können die Falzkanten eingerichtet sein, zwei benachbarte Abschnitte relativ zueinander zu falten bzw. zu knicken, insbesondere um bis zu 180° oder um bis zu 360° (d.h. um bis zu +180° und um bis zu -180°). Durch das Falten bzw. Knicken werden die Abschnitte relativ zueinander gedreht, wobei eine mit der Falzkante zusammenfallende Achse die Drehachse ist. Eine Faltung um bis zu 180° kann eine Faltung um bis zu +180° oder um bis zu -180° sein. Dementsprechend können die Falzkanten als Scharnier und/oder als Doppelscharnier fungieren, um die Abschnitte im gefalteten Zustand des Faltstreifens bestimmungsgemäß anzuordnen.

Der Faltstreifen kann zwei oder mehr Distanzabschnitte aufweisen. Dadurch kann die Stabilität des Faltstreifens im gefalteten Zustand maßgeschneidert bzw. weiter erhöht werden.

Der Faltstreifen kann ein Mittel zum Kleben aufweisen, um zwischen im gefalteten Zustand des Faltstreifens übereinanderliegenden, benachbarten Abschnitten eine stoffschlüssige Verbindung herzustellen. Das Mittel zum Kleben kann ein doppelseitiges Klebeband sein, das mit einer ersten Klebefläche am Faltstreifen befestigt ist, während eine zweite Klebefläche eine Abdeckungsschicht (z.B. Folie) aufweist. Diese Abdeckungsschicht kann unmittelbar vor dem Falten des Faltstreifens entfernt werden, um mittels der zweiten Klebefläche im gefalteten Zustand dann die stoffschlüssige Verbindung herzustellen.

Die Erfindung betrifft weiters einen Testbogen zur Herstellung von zwei oder mehr Vorrichtungen für einen Lateral-Flow-Test, aufweisend zwei oder mehr Faltstreifen gemäß der Erfindung und eine oder mehrere Perforationslinie(n), wobei zwischen jeweils zwei Faltstreifen eine Perforationslinie angeordnet ist. Unter einer Perforationslinie wird eine Reiß- bzw. Trennlinie verstanden, die dazu dient, einen Faltstreifen einfach vom Testbogen abzutrennen. Die Perforationslinie kann eine, insbesondere regelmäßige, Lochung aufweisen. Beispielsweise kann die Perforationslinie elliptische, längliche und/oder runde Löcher aufweisen.

Die Perforationslinie kann an einer Längsseite und/oder an einer Breitseite eines Faltstreifens angeordnet sein. Die Perforationslinie kann sich entlang der gesamten Längsseite und/oder entlang der gesamten Breitseite erstrecken, bzw. nur entlang eines Teils der Längsseite und/oder Breitseite. Erstreckt sich die Perforationslinie nur entlang eines Teils der Längsseite und/oder Breitseite, können benachbarte Faltstreifen entlang des verbleibenden Teils nicht miteinander verbunden sein (etwa durch Vorsehen eines Schnitts zwischen den Faltstreifen an entsprechender Stelle). Damit kann ein einfacheres, schnelleres Abtrennen eines Faltstreifens vom Testbogen erfolgen.

Mit einem Testbogen können mehrere Faltstreifen gleichzeitig hergestellt werden. Zwei oder mehr Faltstreifen können nebeneinander und/oder hintereinander am Testbogen angeordnet sein. Somit ist jeder Faltstreifen je nach Anordnung der Faltstreifen auf dem Testbogen mit einem bis vier weiteren Faltstreifen mittels einer oder mehreren Perforationslinien verbunden. Die Faltstreifen können dann einzeln vom Testbogen abgetrennt und anschließend gefaltet werden. Alternativ kann der Testbogen als Ganzes gefaltet werden, um zwei oder mehr Vorrichtungen herzustellen, die nachfolgend mithilfe der Perforationslinie(n) voneinander getrennt werden können. Ein Testbogen gemäß der Erfindung bietet den Vorteil, dass er platzsparend gelagert und transportiert werden kann. Weiters kann hiermit eine große Anzahl von Vorrichtungen für Lateral-Flow-Tests in kurzer Zeit hergestellt werden.

Die Erfindung betrifft auch ein Verfahren zur Herstellung einer Vorrichtung für einen Lateral-Flow-Test aus dem Faltstreifen, umfassend die Schritte
(a) Bereitstellen des Faltstreifens gemäß der Erfindung,
(b) Befestigen des Teststreifens am Befestigungsbereich, und Vorsehen einer klebefähigen Verbindung auf dem Faltstreifen, und
(c) Falten des Faltstreifens, um den Teststreifen zwischen dem Grundabschnitt und dem Deckabschnitt anzuordnen. Ein weiterer Schritt (d) Fertigstellen der Vorrichtung kann vorgesehen werden.

Der Teststreifen kann in Schritt (b) mit einem Klebstoff und/oder mit einem doppelseitigen Klebeband am Befestigungsbereich befestigt werden. Dann kann er beim nachfolgenden Falten nicht verrutschen und seine Position beibehalten, sodass sichergestellt ist, dass der erste Teil und der zweite Teil des Teststreifens für einen Anwender von außen durch das Applikationsfenster und das Sichtfenster sichtbar bzw. zugänglich sind. Weist der Befestigungsbereich ein Distanzelement auf, kann der Teststreifen am Distanzelement befestigt werden.

Das Vorsehen einer klebefähigen Verbindung auf dem Faltstreifen in Schritt (b) kann das Aufbringen eines Mittels zum Kleben auf den Faltstreifen, insbesondere auf den Grundabschnitt und/oder den Deckabschnitt, umfassen. Das Mittel zum Kleben kann ein doppelseitiges Klebeband und/oder ein Klebstoff sein. Der bereitgestellte Faltstreifen kann bereits ein doppelseitiges Klebeband aufweisen, das mit einer ersten Klebefläche am Faltstreifen befestigt ist, während eine zweite Klebefläche eine Abdeckungsschicht (z.B. Folie) aufweist. In diesem Fall kann Schritt (b) das Entfernen der Abdeckungsschicht vom Klebeband umfassen, um hierdurch eine klebefähige Verbindung vorzusehen.

Weist der Faltstreifen abgesehen vom Grundabschnitt und vom Deckabschnitt einen oder mehrere weitere Abschnitte auf, insbesondere einen oder mehrere Distanzabschnitte, wird die klebefähige Verbindung in Schritt (b) derart vorgesehen, dass im gefalteten Zustand benachbarte (d.h. unmittelbar übereinanderliegende) Abschnitte jeweils stoffschlüssig miteinander verbunden werden können.

In Schritt (c) wird der Faltstreifen derart gefaltet, dass der Teststreifen im gefalteten Zustand des Faltstreifens zwischen dem Grundabschnitt und dem Deckabschnitt angeordnet ist. Weist der Faltstreifen abgesehen vom Grundabschnitt und vom Deckabschnitt einen oder mehrere weitere Abschnitte auf, insbesondere einen oder mehrere Distanzabschnitte, sollte der Faltstreifen in Schritt (c) derart gefaltet werden, dass die weiteren Abschnitte im gefalteten Zustand des Faltstreifens zwischen dem Grundabschnitt und dem Deckabschnitt angeordnet sind. Weist der Faltstreifen einen Distanzabschnitt und einen Fixierabschnitt auf, sollte der Faltstreifen insbesondere derart gefaltet werden, dass der Fixierabschnitt im gefalteten Zustand des Faltstreifens zwischen dem Grundabschnitt und dem Distanzabschnitt angeordnet ist.

Das Fertigstellen der Vorrichtung kann das Herstellen einer stoffschlüssigen Verbindung von im gefalteten Zustand des Faltstreifens benachbarten Abschnitten umfassen. Dies kann durch Aufbringen einer Druckkraft auf die Vorrichtung erreicht werden. Dadurch kann der Faltstreifen verdichtet werden. Somit kann ein auf einer Seite eines der Abschnitte aufgebrachtes Klebeband und/oder aufgebrachter Klebstoff auch gut auf einer dieser Seite zugewandten Seite eines benachbarten Abschnitts anhaften. Das Fertigstellen der Vorrichtung kann auch das Verpacken der Vorrichtung umfassen. Hierdurch kann die Vorrichtung verkaufsfertig gemacht werden.

Das Verfahren kann gleichzeitig für mehrere, insbesondere für alle, auf einem Testbogen angeordneten Faltstreifen durchgeführt werden. Dann kann das Fertigstellen der Vorrichtung weiters das Abtrennen der Vorrichtung vom Testbogen bzw. von einer oder mehreren weiteren Vorrichtungen mittels der Perforationslinie(n) umfassen.

Die Erfindung betrifft auch eine Vorrichtung für einen Lateral-Flow-Test, aufweisend
ein Gehäuse, aufweisend eine Grundschicht und eine Deckschicht, und
einen im Gehäuse aufgenommenen Teststreifen,
wobei die Deckschicht ein Sichtfenster und ein Applikationsfenster aufweist,
wobei ein erster Teil des Teststreifens zwischen der Grundschicht und dem Applikationsfenster und ein zweiter Teil des Teststreifens zwischen der Grundschicht und dem Sichtfenster angeordnet ist, und
wobei die Grundschicht und die Deckschicht mit einer Falzkante miteinander verbunden sind.

Das Gehäuse der Vorrichtung ist einstückig ausgebildet, da die Grundschicht und die Deckschicht mit der Falzkante miteinander verbunden sind. Die Falzkante kann an einer außenliegenden Seite eines Mantels des Gehäuses angeordnet sein, insbesondere an einer Breitseite des Mantels des Gehäuses.

Der Teststreifen kann mit einem Klebstoff und/oder mit einem doppelseitigen Klebeband an einem Befestigungsbereich der Grundschicht befestigt sein. Hierdurch kann er an einem Verrutschen gehindert werden und seine Position relativ zum Gehäuse beibehalten, insbesondere während der Herstellung des Gehäuses mit dem erfindungsgemäßen Verfahren. Im gefalteten Zustand kann der Teststreifen gegebenenfalls durch eine Fixierschicht zusätzlich fixiert werden.

Der Befestigungsbereich der Grundschicht kann ein Distanzelement aufweisen. Der Teststreifen kann dann am Distanzelement befestigt werden. Hierdurch kann die Gefahr eines Kontakts zwischen einer auf den Teststreifen aufgebrachten Probe und der Grundschicht reduziert werden. Das Distanzelement kann zwei oder mehr Distanzstege aufweisen. Hierdurch kann der Teststreifen auf dem Distanzelement im Gleichgewicht gehalten werden. Die Distanzstege können parallel zueinander und parallel zu einer Längsseite oder Breitseite des Faltstreifens sein. Damit kann eine gute Stabilisierung und Befestigung des Teststreifens erreicht werden.

Das Gehäuse kann einen Karton und/oder einen Kunststoff aufweisen. Das Gehäuse kann Zellstoff und/oder Zellulose enthalten. Weist der Faltstreifen einen Karton auf, kann dieser ein Flächengewicht von 200 g/m² oder darüber aufweisen, insbesondere von 300 g/m² oder darüber. Das Gehäuse weist dann eine gute Stabilität auf.

Eine Außenseite und/oder eine Innenseite des Gehäuses kann wasserabweisend sein. Wird die Vorrichtung einer feuchten bzw. nassen Umgebung ausgesetzt, kann eine Beschädigung der Vorrichtung (z.B. Aufweichen, Anlösen) durch eine wasserabweisende Außenseite vermieden werden. Eine wasserabweisende Innenseite kann Störungen bei der Durchführung der Messung vermeiden, z.B. wenn Probenflüssigkeit nicht schnell genug durch den Laufstreifen aufgenommen wird und ein Flüssigkeitstropfen an der Innenseite des Gehäuses nicht durch das Gehäuse aufgesogen werden soll. Die Außenseite und/oder Innenseite des Gehäuses kann beschichtet sein, beispielsweise mit einem hydrophoben Kunststoff, insbesondere mit einem Polyolefin. Eine Beschichtung kann vorteilhaft sein, wenn das Gehäuse einen Karton aufweist. Das Gehäuse kann auch aus einem hydrophoben Kunststoff bestehen. Dann braucht keine zusätzliche Beschichtung vorgesehen zu werden.

Das Gehäuse kann weiters eine Distanzschicht aufweisen, die zwischen der Grundschicht und der Deckschicht angeordnet ist, wobei die Distanzschicht einen Rahmen aufweist, der beabstandet zum Teststreifen, insbesondere zum Laufstreifen des Teststreifens, ist. Die Distanzschicht kann die Stabilität des Gehäuses weiter erhöhen. Auch kann dadurch sichergestellt werden, dass der Laufstreifen des Teststreifens das Gehäuse nicht berührt, da damit die Gefahr verbunden wäre, dass die Probe auf das Distanzelement wandert und eine verschlechterte Messung durchgeführt wird. Die Distanzschicht entspricht dem Distanzabschnitt des Faltstreifens, sodass Merkmale des Distanzabschnitts ebenso auch Merkmale der Distanzschicht sein können.

Das Gehäuse kann weiters eine Fixierschicht aufweisen, die zwischen der Grundschicht und der Deckschicht, und gegebenenfalls zwischen der Grundschicht und der Distanzschicht, angeordnet ist, wobei die Fixierschicht einen Rahmen aufweist, der zumindest teilweise am Teststreifen, insbesondere am Träger des Teststreifens, anliegt. Durch den Fixierabschnitt wird der Teststreifen, insbesondere der Träger, fixiert. Dabei wird der Teststreifen durch den Rahmen des Fixierabschnitts festgehalten. Dadurch kann der Teststreifen im gefalteten Zustand des Faltstreifens auch dann nicht verrutschen, falls er sich von der Befestigung am Befestigungselement löst (z.B. aufgrund von Versagen der Klebeverbindung). Die Fixierschicht entspricht dem Fixierabschnitt des Faltstreifens, sodass Merkmale des Fixierabschnitts ebenso auch Merkmale der Fixierschicht sein können.

Die Vorrichtung kann quaderförmig sein. Eine Längsseite des Teststreifens kann parallel zu einer Längsseite des Gehäuses der Vorrichtung sein. Dadurch können die Abmessungen des zur Herstellung der Vorrichtung verwendeten Faltstreifens möglichst gut ausgenutzt werden, insbesondere, wenn der Faltstreifen einen im Wesentlichen rechteckigen Grundriss hat.

Benachbarte Schichten des Gehäuses können mit einem Klebeband und/oder mit einem Klebstoff miteinander verbunden sein. Insbesondere kann die klebende Verbindung an der Längsseite des Rahmens des Distanzabschnitts und/oder an der Längsseite des Rahmens des Fixierabschnitts vorgesehen werden. Durch die klebende Verbindung wird eine gute Stabilität des Gehäuses erreicht und ein Verrutschen einzelner Schichten gegeneinander vermieden. Die Falzkanten können durch die Verbindung von jeweils zwei Schichten miteinander bereits einen guten Zusammenhalt bereitstellen. Daher braucht an den Seiten der Schichten, an welchen die Falzkanten angeordnet sind, nicht unbedingt eine zusätzliche, klebende Verbindung vorgesehen zu werden.

Die Vorrichtung kann durch Falten eines Faltstreifens gemäß der Erfindung erhalten werden. Hiermit kann die Vorrichtung vergleichsweise einfach und nachhaltig hergestellt werden. Hierbei wird die Grundschicht aus dem Grundabschnitt des Faltstreifens gebildet, die Deckschicht aus dem Deckabschnitt des Faltstreifens, eine gegebenenfalls vorhandene Fixierschicht aus dem Fixierabschnitt des Faltstreifens und eine gegebenenfalls vorhandene Distanzschicht aus dem Distanzabschnitt des Faltstreifens.

Eine Seite des Mantels des Gehäuses kann uneben sein. Unter dieser Seite wird eine außenliegende Seite des Mantels verstanden. Insbesondere kann diese Seite des Mantels Zähne, Kerben und/oder Zacken aufweisen. Daraus wird ersichtlich, dass die Vorrichtung (bzw. der Faltstreifen, aus welchem sie erhalten wurde) von einem Testbogen mittels einer Perforationslinie abgetrennt wurde. Der Mantel des Gehäuse kann auch zwei, drei oder vier unebene Seiten aufweisen. Dies ist abhängig von der Anordnung des Faltstreifens auf dem Testbogen, insbesondere von der Anzahl benachbarter Faltstreifen auf dem Testbogen, mit denen der Faltstreifen mittels Perforationslinien verbunden ist.

Offenbart wird auch die Verwendung der Vorrichtung gemäß der Erfindung zur Durchführung eines Lateral-Flow-Tests.

Für die Zwecke dieser Offenbarung beziehen sich Angaben wie "Grundschicht", "Deckschicht", "Außenseite", "übereinanderliegend", etc. auf die bestimmungsgemäße Ausrichtung des Faltstreifens im gefalteten Zustand bzw. der Vorrichtung.

Die Erfindung betrifft insbesondere die folgenden Ausführungsformen:
1. Faltstreifen zur Herstellung einer Vorrichtung für einen Lateral-Flow-Test, aufweisend
   einen Grundabschnitt und einen Deckabschnitt,
   eine in einem ungefalteten Zustand des Faltstreifens zwischen dem Grundabschnitt und dem Deckabschnitt angeordnete Falzkante,
   wobei der Grundabschnitt einen Befestigungsbereich zur Befestigung eines Teststreifens aufweist,
   wobei der Deckabschnitt ein Sichtfenster und ein Applikationsfenster aufweist, und
   wobei in einem gefalteten Zustand des Faltstreifens ein erster Teil des Befestigungsbereichs zwischen dem Grundabschnitt und dem Applikationsfenster und ein zweiter Teil des Befestigungsbereichs zwischen dem Grundabschnitt und dem Sichtfenster angeordnet ist.
2. Faltstreifen nach Ausführungsform 1, weiters aufweisend einen am Befestigungsbereich befestigten Teststreifen, wobei im gefalteten Zustand des Faltstreifens ein erster Teil des Teststreifens zwischen dem Grundabschnitt und dem Applikationsfenster und ein zweiter Teil des Teststreifens zwischen dem Grundabschnitt und dem Sichtfenster angeordnet ist.
3. Faltstreifen nach Ausführungsform 2, wobei der Teststreifen einen Träger zur Befestigung am Befestigungsbereich aufweist, und wobei der Teststreifen einen am Träger befestigten Laufstreifen aufweist, vorzugsweise wobei der Laufstreifen einen Applikationsbereich und einen Ergebnisbereich aufweist.
4. Faltstreifen nach einer der Ausführungsformen 1 bis 3, wobei der Faltstreifen einen im Wesentlichen rechteckigen Grundriss hat, und/oder wobei die Falzkante normal zu einer Längsseite des Faltstreifens ist, und/oder wobei die Falzkante eine Vertiefung aufweist, insbesondere eine Rille und/oder eine Nut.
5. Faltstreifen nach einer der Ausführungsformen 1 bis 4, wobei der Befestigungsbereich des Grundabschnitts ein Distanzelement aufweist, das zur Befestigung des Teststreifens eingerichtet ist.
6. Faltstreifen nach Ausführungsform 5, wobei das Distanzelement zwei oder mehr Distanzstege aufweist.
7. Faltstreifen nach Ausführungsform 6, wobei die Distanzstege parallel zueinander und parallel zu einer Längsseite oder zu einer Breitseite des Faltstreifens sind.
8. Faltstreifen nach einer der Ausführungsformen 2 bis 7, wobei der Teststreifen mit einem Klebstoff und/oder mit einem doppelseitigen Klebeband am Befestigungsbereich befestigt ist.
9. Faltstreifen nach einer der Ausführungsformen 1 bis 8, wobei der Faltstreifen einen Karton und/oder einen Kunststoff enthält.
10. Faltstreifen nach Ausführungsform 10, wobei der Karton ein Flächengewicht von 200 g/m² oder mehr aufweist, insbesondere von 300 g/m² oder mehr.
11. Faltstreifen nach einer der Ausführungsformen 1 bis 10, wobei eine im gefalteten Zustand außenliegende Seite des Grundabschnitts und/oder des Deckabschnitts wasserabweisend ist.
12. Faltstreifen nach einer der Ausführungsformen 9 bis 11, wobei der Faltstreifen aus einem Kunststoff besteht, vorzugsweise aus einem hydrophoben Kunststoff.
13. Faltstreifen nach einer der Ausführungsformen 1 bis 12, wobei eine Seitenfläche des Faltstreifens Zähne, Kerben und/oder Zacken aufweist.
14. Faltstreifen nach einer der Ausführungsformen 1 bis 13, weiters aufweisend einen Distanzabschnitt, der im gefalteten Zustand des Faltstreifens zwischen dem Grundabschnitt und dem Deckabschnitt angeordnet ist, wobei der Distanzabschnitt einen Rahmen aufweist, der vorzugsweise eingerichtet ist, im gefalteten Zustand des Faltstreifens beabstandet zum Teststreifen, insbesondere zum Laufstreifen des Teststreifens, zu sein.
15. Faltstreifen nach Ausführungsform 14,
   wobei der Distanzabschnitt im ungefalteten Zustand des Faltstreifens zwischen dem Grundabschnitt und dem Deckabschnitt angeordnet ist, wobei je eine Falzkante zwischen dem Grundabschnitt und dem Distanzabschnitt sowie zwischen dem Distanzabschnitt und dem Deckabschnitt angeordnet ist, oder
   wobei der Grundabschnitt im ungefalteten Zustand des Faltstreifens zwischen dem Distanzabschnitt und dem Deckabschnitt angeordnet ist, wobei je eine Falzkante zwischen dem Distanzabschnitt und dem Grundabschnitt sowie zwischen dem Grundabschnitt und dem Deckabschnitt angeordnet ist.
16. Faltstreifen nach einer der Ausführungsformen 1 bis 15, weiters aufweisend einen Fixierabschnitt, der im gefalteten Zustand des Faltstreifens zwischen dem Grundabschnitt und dem Deckabschnitt angeordnet ist, wobei der Fixierabschnitt einen Rahmen aufweist, der vorzugsweise eingerichtet ist, im gefalteten Zustand des Faltstreifens zumindest teilweise am Teststreifen, insbesondere am Träger des Teststreifens, anzuliegen.
17. Faltstreifen nach Ausführungsform 16,
   wobei der Fixierabschnitt im ungefalteten Zustand des Faltstreifens zwischen dem Grundabschnitt und dem Deckabschnitt angeordnet ist, wobei je eine Falzkante zwischen dem Grundabschnitt und dem Fixierabschnitt sowie zwischen dem Fixierabschnitt und dem Deckabschnitt angeordnet ist, oder
   wobei der Grundabschnitt im ungefalteten Zustand des Faltstreifens zwischen dem Fixierabschnitt und dem Deckabschnitt angeordnet ist, wobei je eine Falzkante zwischen dem Fixierabschnitt und dem Grundabschnitt sowie zwischen dem Grundabschnitt und dem Fixierabschnitt angeordnet ist.
18. Faltstreifen nach Ausführungsform 16 oder 17, wobei eine Längsseite des Rahmens des Fixierabschnitts einen Teil aufweist, der breiter ist als eine Längsseite des Rahmens des Distanzabschnitts, und/oder wobei die Ausnehmung, die durch den Rahmen des Fixierabschnitts gebildet wird, an einem Teil ihrer Längsseite schmäler als die durch den Rahmen des Distanzabschnitts gebildete Ausnehmung an ihrer Längsseite.
19. Faltstreifen nach einer der Ausführungsformen 14 bis 18, aufweisend zwei oder mehr Distanzabschnitte.
20. Faltstreifen nach einer der Ausführungsformen 16 bis 19, aufweisend den Grundabschnitt, den Deckabschnitt, den Distanzabschnitt und den Fixierabschnitt, wobei im ungefalteten Zustand des Faltstreifens jeweils eine Falzkante zwischen den Abschnitten angeordnet ist, vorzugsweise wobei im gefalteten Zustand des Faltstreifens der Fixierabschnitt zwischen dem Grundabschnitt und dem Distanzabschnitt angeordnet ist, und wobei der Distanzabschnitt zwischen dem Fixierabschnitt und dem Deckabschnitt angeordnet ist.
21. Faltstreifen nach einer der Ausführungsformen 1 bis 20, wobei die Falzkante eingerichtet ist, zwei benachbarte Abschnitte relativ zueinander zu falten oder zu knicken, vorzugsweise um bis zu 180° oder um bis zu 360°.
22. Faltstreifen nach einer der Ausführungsformen 1 bis 21, aufweisend ein Mittel zum Kleben, um zwischen im gefalteten Zustand des Faltstreifens übereinanderliegenden, benachbarten Abschnitten eine stoffschlüssige Verbindung herzustellen.
23. Testbogen zur Herstellung von zwei oder mehr Vorrichtungen für einen Lateral-Flow-Test, aufweisend zwei oder mehr Faltstreifen nach einer der Ausführungsformen 1 bis 22, und eine oder mehrere Perforationslinie(n), wobei zwischen jeweils zwei Faltstreifen eine Perforationslinie angeordnet ist.
24. Verfahren zur Herstellung einer Vorrichtung für einen Lateral-Flow-Test aus einem Faltstreifen, insbesondere zur Herstellung einer Vorrichtung nach einer der Ausführungsformen 28 bis 42, umfassend die Schritte
   (a) Bereitstellen des Faltstreifens gemäß einer der Ausführungsformen 1 bis 22,
   (b) Befestigen des Teststreifens am Befestigungsbereich des Grundabschnitts, und Vorsehen einer klebefähigen Verbindung auf dem Faltstreifen,
   (c) Falten des Faltstreifens, um den Teststreifen zwischen dem Grundabschnitt und dem Deckabschnitt anzuordnen.
25. Verfahren nach Ausführungsform 24, wobei das Vorsehen einer klebefähigen Verbindung auf dem Faltstreifen in Schritt (b) das Aufbringen eines Mittels zum Kleben auf den Grundabschnitt und/oder den Deckabschnitt umfasst.
26. Verfahren nach Ausführungsform 25, wobei das Mittel zum Kleben ein doppelseitiges Klebeband und/oder ein Klebstoff ist.
27. Verfahren nach einer der Ausführungsformen 24 bis 26, mit dem zusätzlichen Schritt (d) des Fertigstellens der Vorrichtung, vorzugsweise wobei Schritt (d) das Herstellen einer stoffschlüssigen Verbindung von im gefalteten Zustand benachbarten Abschnitten beinhaltet.
28. Vorrichtung für einen Lateral-Flow-Test, insbesondere erhältlich durch Falten des Faltstreifens nach einem der Ausführungsformen 1 bis 22, aufweisend
   ein Gehäuse, aufweisend eine Grundschicht und eine Deckschicht, und
   einen im Gehäuse aufgenommenen Teststreifen für einen Lateral-Flow-Test,
   wobei die Deckschicht ein Sichtfenster und ein Applikationsfenster aufweist,
   wobei ein erster Teil des Teststreifens zwischen der Grundschicht und dem Applikationsfenster und ein zweiter Teil des Teststreifens zwischen der Grundschicht und dem Sichtfenster angeordnet ist, und
   wobei die Grundschicht und die Deckschicht mit einer Falzkante miteinander verbunden sind.
29. Vorrichtung nach Ausführungsform 28, wobei der Teststreifen mit einem Klebstoff und/oder mit einem doppelseitigen Klebeband an einem Befestigungsbereich der Grundschicht befestigt ist.
30. Vorrichtung nach Ausführungsform 28 oder 29, wobei der Befestigungsbereich der Grundschicht ein Distanzelement aufweist, an welchem der Teststreifen befestigt ist.
31. Vorrichtung nach einer der Ausführungsformen 28 bis 30, wobei das Gehäuse einen Karton und/oder einen Kunststoff aufweist.
32. Vorrichtung nach einer der Ausführungsformen 28 bis 31, wobei der Karton ein Flächengewicht von 200 g/m² oder mehr aufweist, insbesondere von 300 g/m² oder mehr.
33. Vorrichtung nach einer der Ausführungsformen 28 bis 32, wobei eine Außenseite und/oder eine Innenseite des Gehäuses wasserabweisend ist.
34. Vorrichtung nach einer der Ausführungsformen 31 bis 33, wobei das Gehäuse aus einem Kunststoff besteht, vorzugsweise aus einem hydrophoben Kunststoff.
35. Vorrichtung nach einer der Ausführungsformen 28 bis 34, wobei das Gehäuse weiters eine Distanzschicht aufweist, die zwischen der Grundschicht und der Deckschicht angeordnet ist, und wobei die Distanzschicht einen Rahmen aufweist, der vorzugsweise zum Teststreifen, insbesondere zum Laufstreifen des Teststreifens, beabstandet ist.
36. Vorrichtung nach einer der Ausführungsformen 28 bis 35, wobei das Gehäuse weiters eine Fixierschicht aufweist, die zwischen der Grundschicht und der Deckschicht, und gegebenenfalls zwischen der Grundschicht und der Distanzschicht angeordnet ist, wobei die Fixierschicht einen Rahmen aufweist, der vorzugsweise zumindest teilweise am Teststreifen, insbesondere am Träger des Teststreifens, anliegt.
37. Vorrichtung nach Ausführungsform 36, wobei eine Längsseite des Rahmens des Fixierabschnitts an ihrer Innenkante wellenförmig ist.
38. Vorrichtung nach einer der Ausführungsformen 28 bis 37, wobei die Vorrichtung quaderförmig ist.
39. Vorrichtung nach einer der Ausführungsformen 28 bis 38, wobei eine Längsseite des Teststreifens parallel zu einer Längsseite des Faltstreifens ist.
40. Vorrichtung nach einer der Ausführungsformen 28 bis 39, wobei benachbarte Schichten des Gehäuses mit einem Klebeband und/oder mit einem Klebstoff miteinander verbunden sind.
41. Vorrichtung nach einer der Ausführungsformen 28 bis 40, erhältlich durch das Verfahren nach einer der Ausführungsformen 24 bis 27.
42. Vorrichtung nach einer der Ausführungsformen 28 bis 41, wobei eine Seite eines Mantels des Gehäuses Zähne, Kerben und/oder Zacken aufweist.
43. Verwendung der Vorrichtung nach einer der Ausführungsformen 28 bis 42 zur Durchführung eines Lateral-Flow-Tests.

Die Erfindung wird nachstehend anhand von Figurenbeschreibungen einiger Ausführungsformen weiter erläutert. Diese Ausführungsformen sind besonders bevorzugte Ausführungsbeispiele, auf die die Erfindung jedoch nicht beschränkt sein soll.
Fig. 1 zeigt einen Faltstreifen zur Herstellung einer Vorrichtung für einen Lateral-Flow-Test in einem ungefalteten Zustand.
Fig. 2 zeigt den Faltstreifen der Fig. 1, weiters aufweisend einen Teststreifen.
Fig. 3 zeigt eine Vorrichtung für einen Lateral-Flow-Test, erhalten durch Falten des Faltstreifens der Fig. 2.
Fig. 4 zeigt einen Testbogen, der nebeneinander angeordnete Faltstreifen in einem ungefalteten Zustand aufweist, die jeweils dem Faltstreifen der Fig. 1 entsprechen.
Fig. 5 zeigt den Testbogen der Fig. 4, wobei jeder Faltstreifen einen Teststreifen aufweist.

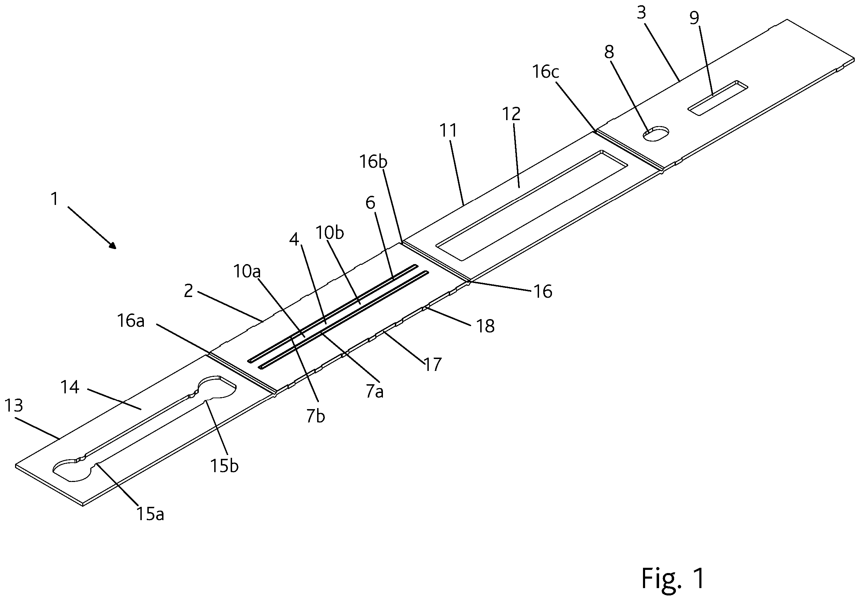

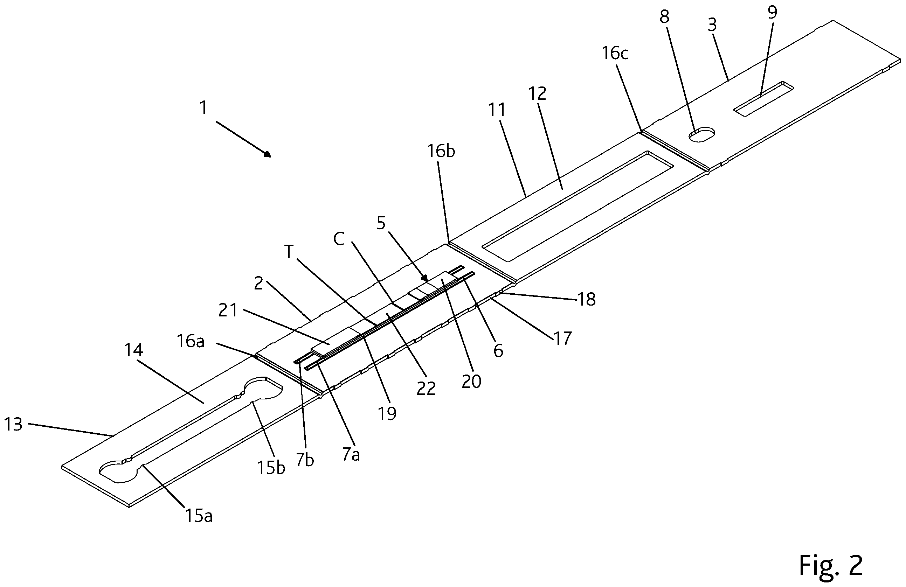

In Fig. 1 ist ein Faltstreifen 1 mit einem im Wesentlichen rechteckigen Grundriss in einem ungefalteten Zustand dargestellt. Der Faltstreifen 1 weist einen Grundabschnitt 2 und einen Deckabschnitt 3 auf. Der Grundabschnitt 2 weist einen Befestigungsbereich 4 zur Befestigung eines Teststreifens 5 (dargestellt in Fig. 2) auf. Der Befestigungsbereich 4 weist ein Distanzelement 6 mit zwei zueinander parallelen Distanzstegen 7a, 7b auf.

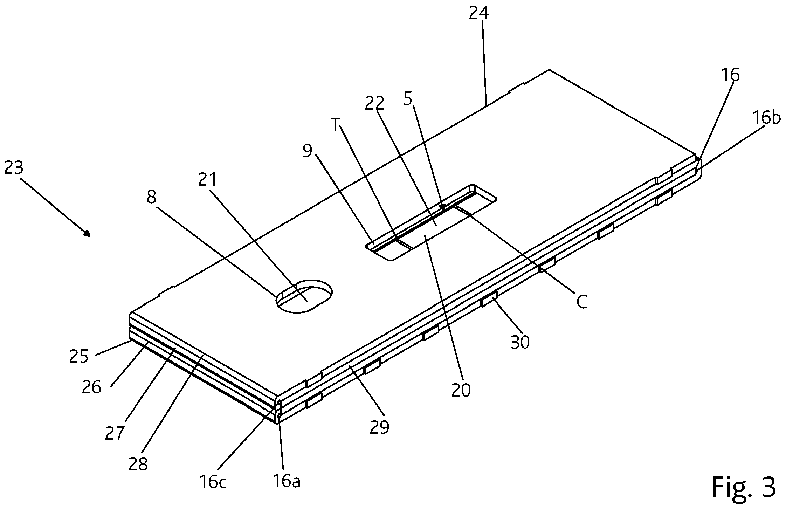

Weiters aus Fig. 1 ist ersichtlich, dass der Deckabschnitt 3 ein Applikationsfenster 8 und ein Sichtfenster 9 aufweist. In einem gefalteten Zustand des Faltstreifens 1 ist ein erster Teil 10a des Befestigungsbereichs 4 zwischen dem Grundabschnitt 2 und dem Applikationsfenster 8 und ein zweiter Teil 10b des Befestigungsbereichs 4 zwischen dem Grundabschnitt 2 und dem Sichtfenster 9 angeordnet (vgl. Fig. 3).

Wie aus Fig. 1 auch ersichtlich ist, weist der Faltstreifen 1 einen Distanzabschnitt 11 auf, der im ungefalteten Zustand des Faltstreifens 1 zwischen dem Grundabschnitt 2 und dem Deckabschnitt 3 angeordnet ist. Der Distanzabschnitt 11 weist einen Rahmen 12 auf, der eingerichtet ist, im gefalteten Zustand des Faltstreifens 1 beabstandet zum Teststreifen 5, insbesondere zu einem Laufstreifen 20 des Teststreifens 5 (dargestellt in Fig. 2), zu sein. Durch den Rahmen 12 wird eine rechteckige Ausnehmung im Distanzabschnitt 11 gebildet. Der Rahmen 12 ist durch ein umlaufendes Profil gebildet und weist vier Seiten auf (zwei Breitseiten und zwei Längsseiten), die eingerichtet sind, im gefalteten Zustand des Faltstreifens 1 beabstandet zum Teststreifen 5 zu sein.

Der Faltstreifen 1 gemäß Fig. 1 weist auch einen Fixierabschnitt 13 auf, wobei der Grundabschnitt 2 im ungefalteten Zustand des Faltstreifens 1 zwischen dem Fixierabschnitt 13 und dem Deckabschnitt 3 angeordnet ist. Der Fixierabschnitt 13 weist einen durch ein umlaufendes Profil gebildeten Rahmen 14 auf, der vier Seiten aufweist (zwei Breitseiten und zwei Längsseiten). Beide Längsseiten des Rahmens 14 sind an ihren Innenkanten wellenförmig und weisen jeweils zwei Teile 15a, 15b mit jeweils zwei Wellenbergen auf. Die Teile 15a, 15b sind eingerichtet, im gefalteten Zustand des Faltstreifens 1 in endnahen Bereichen des Teststreifens 5 mit ihren Wellenbergen an einem Träger 19 des Teststreifens 5 (dargestellt in Fig. 2) anzuliegen. Dadurch kann der Teststreifen 5 im gefalteten Zustand des Faltstreifens 1 in einer durch den Rahmen 14 gebildeten Ausnehmung fixiert werden. Die Teile 15a, 15b des Rahmens 14 sind breiter als die Längsseiten des Rahmens 12 des Distanzabschnitts 11. Die Ausnehmung, die durch den Rahmen 14 des Fixierabschnitts 13 gebildet wird, ist an den breiten Teilen 15a, 15b schmäler als die durch den Rahmen 12 des Distanzabschnitts 11 gebildete Ausnehmung entlang deren gesamter Längsseite. Die breiten Teile 15a, 15b der Längsseite des Rahmens 14 des Fixierabschnitts 13 korrespondieren mit schmalen Teilen der Längsseite des durch den Rahmen 14 des Fixierabschnitts 13 gebildeten Ausnehmung. Dementsprechend hat der Fixierabschnitt 13 einen rechteckigen Grundriss und kann im gefalteten Zustand des Faltstreifens 1 bündig mit den anderen Abschnitten 2, 3, 11 des Faltstreifens 1 sein.

Wie aus Fig. 1 weiters ersichtlich ist, sind zwischen den einzelnen Abschnitten Falzkanten 16 vorgesehen, die eine Rille aufweisen und normal zu einer Längsseite des Faltstreifens 1 sind. Konkret ist zwischen dem Fixierabschnitt 13 und dem Grundabschnitt 2, zwischen dem Grundabschnitt 2 und dem Distanzabschnitt 11 sowie zwischen dem Distanzabschnitt 11 und dem Deckabschnitt 3 jeweils eine Falzkante 16a-c vorgesehen. Die Falzkanten 16a-c sind eingerichtet, die Abschnitte 2, 3, 11, 13 derart zu falten, um sie übereinanderliegend anzuordnen und eine Vorrichtung 23 gemäß der Erfindung herzustellen (dargestellt in Fig. 2).

Fig. 1 zeigt weiters, dass eine Seitenfläche 17 des Faltstreifens 1 Zähne 18 aufweist. Dies lässt erkennen, dass der Faltstreifen 1 von einem Testbogen 31 (dargestellt in Fig. 3) abgetrennt wurde.

Fig. 2 zeigt den Faltstreifen 1 der Fig. 1, weiters aufweisend einen Teststreifen 5 mit einem rechteckigen Grundriss. Eine Längsseite des Teststreifens 5 ist parallel zu einer Längsseite des Faltstreifens 1. Der Teststreifen 5 weist einen Träger 19 auf, mit welchem er am Befestigungsbereich 4 des Grundabschnitts 2 (siehe Fig. 1) befestigt ist. Auf dem Träger 19 ist ein Laufstreifen 20 zur Aufnahme und Messung einer Probe befestigt, der einen Applikationsbereich 21 aufweist, auf welchen die Probe aufgebracht werden kann, und einen Ergebnisbereich 22, in welchem sich eine Kontrolllinie (C) ausbilden kann, und im Falle eines positiven Tests weiters eine Testlinie (T). Der Teststreifen 5 ist derart am Befestigungsbereich 4 befestigt, dass ein erster Teil des Teststreifens 5 im gefalteten Zustand des Faltstreifens 1 zwischen dem Grundabschnitt 2 und dem Applikationsfenster 8 angeordnet ist, und dass ein zweiter Teil des Teststreifens 5 im gefalteten Zustand des Faltstreifens 1 zwischen dem Grundabschnitt 2 und dem Sichtfenster 9 angeordnet ist. Dabei weist der erste Teil des Teststreifens 5 den Applikationsbereich 21 auf und der zweite Teil des Teststreifens 5 den Ergebnisbereich 22.

Fig. 3 zeigt eine quaderförmige Vorrichtung 23 für einen Lateral-Flow-Test. Diese wurde durch Falten des Faltstreifens 1 der Fig. 2 erhalten. Die Vorrichtung 23 umfasst ein Gehäuse 24, aufweisend eine Grundschicht 25, eine Fixierschicht 26, eine Distanzschicht 27 und eine Deckschicht 28, die dem Grundabschnitt 2, dem Fixierabschnitt 13, dem Distanzabschnitt 11 und dem Deckabschnitt 3 des Faltstreifens 1 entsprechen (vgl. Fig. 2). Das Gehäuse 24 ist einstückig ausgebildet, wobei die Schichten 25-28 mit Falzkanten 16a-c miteinander verbunden sind. Die Falzkanten 16a-c sind dabei an Breitseiten des Mantels des Gehäuses 24 angeordnet.

Weiters ist aus Fig. 3 ersichtlich, dass der Teststreifen 5 im Gehäuse 24 aufgenommen und an einem Befestigungsbereich 4 der Grundschicht 25 derart befestigt ist, dass eine Längsseite des Teststreifens 5 parallel zu einer Längsseite des Gehäuses 24 ist. Der erste Teil des Teststreifens 5, der den Applikationsbereich 21 aufweist, ist durch das Applikationsfenster 8 für einen Anwender von außen zugänglich. Der zweite Teil des Teststreifens 5, der den Ergebnisbereich 22 aufweist, kann durch das Sichtfenster 9 vom Anwender von außen eingesehen werden. Im Ergebnisbereich 22 kann sich eine Kontrolllinie (C) ausbilden, und im Falle eines positiven Tests weiters eine Testlinie (T). Eine Seite 29 des Mantels des Gehäuses 24 weist Zähne 30 auf. Dies lässt erkennen, dass die Vorrichtung 23 bzw. der Faltstreifen 1 von einem Testbogen 31 (dargestellt in Fig. 3) abgetrennt wurde.

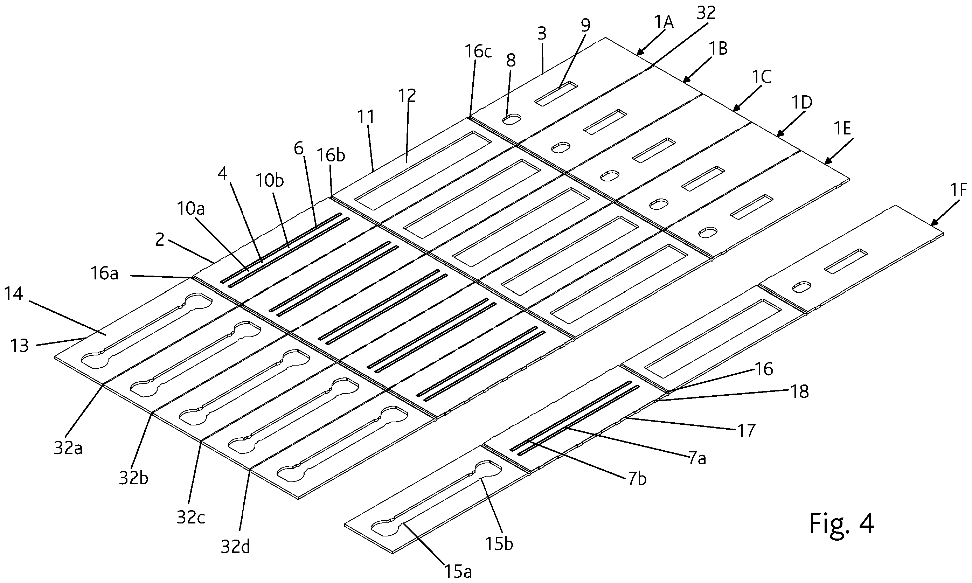

In Fig. 4 ist ein Testbogen 31 dargestellt, der sechs nebeneinander angeordnete Faltstreifen 1A-F aufweist, die jeweils dem in Fig. 1 beschriebenen Faltstreifen 1 entsprechen. Zwischen den Faltstreifen 1A-F sind Perforationslinien 32 angeordnet. Konkret sind an Längsseiten der Faltstreifen 1A-E die Perforationslinien 32a-d angeordnet, wobei jeweils eine der Perforationslinien 32a-d zwischen zwei benachbarten Faltstreifen 1A-E vorgesehen ist. Der Faltstreifen 1F wurde in der Darstellung der Fig. 4 bereits vom Testbogen 31 abgetrennt, um ihn nachfolgend zu falten und eine Vorrichtung 23 (dargestellt in Fig. 3) für einen Lateral-Flow-Test herzustellen.

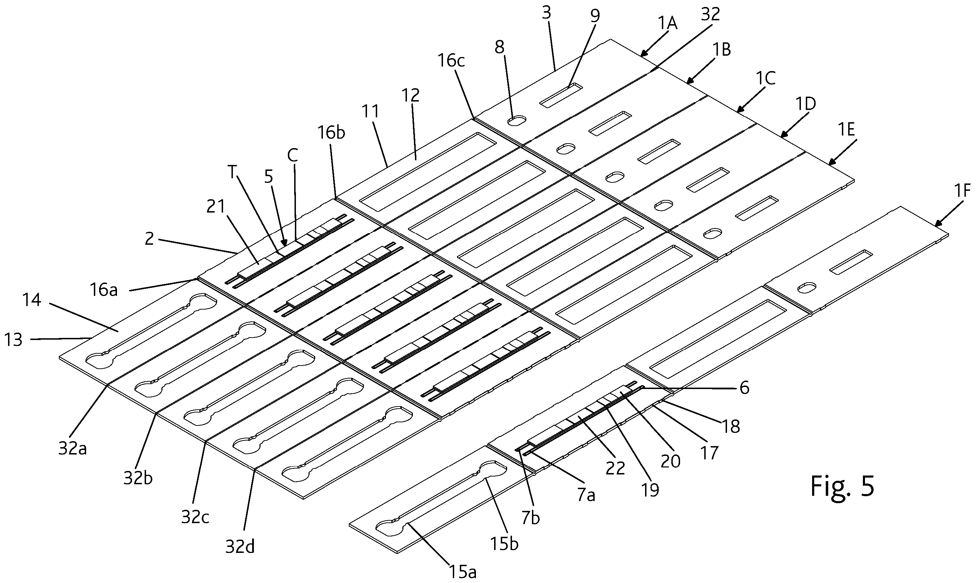

Fig. 5 zeigt den Testbogen 31 der Fig. 4, wobei jeder Faltstreifen 1A-F einen Teststreifen 4 aufweist, der an den zwei Distanzstegen 7a, 7b des Distanzelements 6 befestigt ist. Jeder Faltstreifen 1A-F entspricht somit dem in Fig. 2 gezeigten Faltstreifen 1.

## Patentansprüche

1. Faltstreifen (1, 1A-F) zur Herstellung einer Vorrichtung (23) für einen Lateral-Flow-Test, aufweisend
einen Grundabschnitt (2) und einen Deckabschnitt (3),
eine in einem ungefalteten Zustand des Faltstreifens (1, 1A-F) zwischen dem Grundabschnitt (2) und dem Deckabschnitt (3) angeordnete Falzkante (16, 16a-c),
wobei der Grundabschnitt (2) einen Befestigungsbereich (4) zur Befestigung eines Teststreifens (5) aufweist,
wobei der Deckabschnitt (3) ein Sichtfenster (9) und ein Applikationsfenster (8) aufweist, und
wobei in einem gefalteten Zustand des Faltstreifens (1, 1A-F) ein erster Teil (10a) des Befestigungsbereichs (4) zwischen dem Grundabschnitt (2) und dem Applikationsfenster (8) und ein zweiter Teil (10b) des Befestigungsbereichs (4) zwischen dem Grundabschnitt (2) und dem Sichtfenster (9) angeordnet ist.

2. Faltstreifen (1, 1A-F) nach Anspruch 1, weiters aufweisend einen am Befestigungsbereich (4) befestigten Teststreifen (5), wobei im gefalteten Zustand des Faltstreifens (1) ein erster Teil des Teststreifens (5) zwischen dem Grundabschnitt (2) und dem Applikationsfenster (8) und ein zweiter Teil des Teststreifens (5) zwischen dem Grundabschnitt (2) und dem Sichtfenster (9) angeordnet ist.

3. Faltstreifen (1, 1A-F) nach Anspruch 1 oder 2, wobei eine Seitenfläche (17) des Faltstreifens (1, 1A-F) Zähne (18), Kerben und/oder Zacken aufweist.

4. Faltstreifen (1, 1A-F) nach einem der Ansprüche 1 bis 3, weiters aufweisend einen Distanzabschnitt (11), der im gefalteten Zustand des Faltstreifens (1, 1A-F) zwischen dem Grundabschnitt (2) und dem Deckabschnitt (3) angeordnet ist, wobei der Distanzabschnitt (11) einen Rahmen (12) aufweist, der vorzugsweise eingerichtet ist, im gefalteten Zustand des Faltstreifens (1, 1A-F) beabstandet zum Teststreifen (5) zu sein.

5. Faltstreifen nach Anspruch 4,
wobei der Distanzabschnitt (11) im ungefalteten Zustand des Faltstreifens (1) zwischen dem Grundabschnitt (2) und dem Deckabschnitt (3) angeordnet ist, wobei je eine Falzkante zwischen dem Grundabschnitt und dem Distanzabschnitt sowie zwischen dem Distanzabschnitt und dem Deckabschnitt angeordnet ist, oder
wobei der Grundabschnitt (2) im ungefalteten Zustand des Faltstreifens (1) zwischen dem Distanzabschnitt (11) und dem Deckabschnitt (3) angeordnet ist, wobei je eine Falzkante zwischen dem Distanzabschnitt und dem Grundabschnitt sowie zwischen dem Grundabschnitt und dem Deckabschnitt angeordnet ist.

6. Faltstreifen nach einem der Ansprüche 1 bis 5, weiters aufweisend einen Fixierabschnitt (13), der im gefalteten Zustand des Faltstreifens (1) zwischen dem Grundabschnitt (2) und dem Deckabschnitt (3) angeordnet ist, wobei der Fixierabschnitt (13) einen Rahmen aufweist, der eingerichtet ist, im gefalteten Zustand des Faltstreifens zumindest teilweise am Teststreifen, insbesondere am Träger des Teststreifens, anzuliegen.

7. Faltstreifen nach Anspruch 6,
wobei der Fixierabschnitt (13) im ungefalteten Zustand des Faltstreifens (1) zwischen dem Grundabschnitt (2) und dem Deckabschnitt (3) angeordnet ist, wobei je eine Falzkante zwischen dem Grundabschnitt und dem Fixierabschnitt sowie zwischen dem Fixierabschnitt und dem Deckabschnitt angeordnet ist, oder
wobei der Grundabschnitt (2) im ungefalteten Zustand des Faltstreifens (1) zwischen dem Fixierabschnitt (13) und dem Deckabschnitt (3) angeordnet ist, wobei je eine Falzkante zwischen dem Fixierabschnitt und dem Grundabschnitt sowie zwischen dem Grundabschnitt und dem Fixierabschnitt angeordnet ist.

8. Faltstreifen nach Anspruch 4 in Kombination mit Anspruch 6, aufweisend den Grundabschnitt (2), den Deckabschnitt (3), den Distanzabschnitt (11) und den Fixierabschnitt (13), wobei im ungefalteten Zustand des Faltstreifens jeweils eine Falzkante zwischen den Abschnitten angeordnet ist, vorzugsweise wobei im gefalteten Zustand des Faltstreifens der Fixierabschnitt zwischen dem Grundabschnitt und dem Distanzabschnitt angeordnet ist, und wobei der Distanzabschnitt zwischen dem Fixierabschnitt und dem Deckabschnitt angeordnet ist.

9. Testbogen (31) zur Herstellung von zwei oder mehr Vorrichtungen (23) für einen Lateral-Flow-Test, aufweisend
zwei oder mehr Faltstreifen (1, 1A-F) nach einem der Ansprüche 1 bis 8, und
eine oder mehrere Perforationslinie(n) (32, 32a-d),
wobei zwischen jeweils zwei Faltstreifen (1, 1A-F) eine Perforationslinie (32, 32a-d) angeordnet ist.

10. Verfahren zur Herstellung einer Vorrichtung (23) für einen Lateral-Flow-Test aus einem Faltstreifen (1, 1A-F), umfassend die Schritte
(a) Bereitstellen des Faltstreifens (1, 1A-F) nach einem der Ansprüche 1 bis 8,
(b) Befestigen des Teststreifens (5) am Befestigungsbereich (4), und Vorsehen einer klebefähigen Verbindung auf dem Faltstreifen (1, 1A-F), und
(c) Falten des Faltstreifens (1, 1A-F), um den Teststreifen (5) zwischen dem Grundabschnitt (2) und dem Deckabschnitt (3) anzuordnen.

11. Vorrichtung (23) für einen Lateral-Flow-Test, aufweisend
ein Gehäuse (24), aufweisend eine Grundschicht (25) und eine Deckschicht (28), und
einen im Gehäuse (24) aufgenommenen Teststreifen (5),
wobei die Deckschicht (28) ein Sichtfenster (9) und ein Applikationsfenster (8) aufweist,
wobei ein erster Teil des Teststreifens (5) zwischen der Grundschicht (25) und dem Applikationsfenster (8) und ein zweiter Teil des Teststreifens (5) zwischen der Grundschicht (25) und dem Sichtfenster (9) angeordnet ist, und
wobei die Grundschicht (25) und die Deckschicht (28) mit einer Falzkante (16, 16a-c) miteinander verbunden sind.

12. Vorrichtung (23) nach Anspruch 11, wobei der Befestigungsbereich (4) der Grundschicht (25) ein Distanzelement (6) aufweist.

13. Vorrichtung (23) nach Anspruch 11 oder 12, wobei das Gehäuse (24) weiters eine Distanzschicht (27) aufweist, die zwischen der Grundschicht (25) und der Deckschicht (28) angeordnet ist, und wobei die Distanzschicht (27) einen Rahmen (12) aufweist, der vorzugsweise beabstandet zum Teststreifen (5) ist.

14. Vorrichtung (23) nach einem der Ansprüche 11 bis 13, wobei das Gehäuse (24) weiters eine Fixierschicht (26) aufweist, die zwischen der Grundschicht (25) und der Deckschicht (28), und gegebenenfalls zwischen der Grundschicht (25) und einer Distanzschicht (27), angeordnet ist, wobei die Fixierschicht (26) einen Rahmen (14) aufweist, der zumindest teilweise am Teststreifen (5) anliegt.

15. Verwendung der Vorrichtung nach einem der Ansprüche 11 bis 14 zur Durchführung eines Lateral-Flow-Tests.
